(19) Europäisches Patentamt
European Patent Office
Office européen des brevets

(11) **EP 2 016 896 A1**

(12) **EUROPEAN PATENT APPLICATION**

(43) Date of publication:
**21.01.2009 Bulletin 2009/04**

(51) Int Cl.:
***A61B 5/053*** (2006.01)

(21) Application number: **08157817.1**

(22) Date of filing: **06.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **11.06.2007 JP 2007153694**

(71) Applicant: **TANITA CORPORATION Tokyo 174-8630 (JP)**

(72) Inventor: **Okura, Masashi 14-2, Maenocho 1-chome Itabashi-ku Tokyo 174-8630 (JP)**

(74) Representative: **Grünecker, Kinkeldey, Stockmair & Schwanhäusser Anwaltssozietät Leopoldstrasse 4 80802 München (DE)**

(54) **A muscular volume balance evaluation apparatus, a muscular volume balance valuation**

(57)    The muscular volume balance measuring apparatus that measures the balance of the muscular volume in a specific part is provided.

At least 2 sets of current applying method, which inserts a specific part of the body and applies current to living bodies, or a voltmeter, which measures voltage, are provided.

It also provides a bioelectricity impedance measuring method which measures bioelectricity impedance of a measured region from applied current and measured

voltage. It also has a muscular volume calculating method which computes muscular volume from measured bioelectricity impedance.

Further, balance of muscular volume of the inserted part is calculated from each set of the computed the muscular volume.

In addition, there is a feature of being able to expand and contract for the longitudinal width, which is the distance which inserts a specific part, and breadth, which is the distance between current applying method or between the voltmeter.

Fig. 1

1

EP 2 016 896 A1

**Description**

BACKGROUND OF THE INVENTION

[Field of the Invention]

Especially, this invention relates to a muscular volume balance evaluation apparatus and a muscular volume balance valuation method.

[Background of the Invention]

By advance of aging of recently, it has been a problem that elderly people's fall accident is increasing.
When elderly people fall once and suffer a fracture, elderly people who has low healing capacity stay bedridden in many cases, and causing a problem of a lowered of QOL (quality of life) of old age.
As for a part of a cause which elderly people tumble, it is known that one of the factors is deminution of the muscular power of elderly people by aging.
The reason why muscular power declines is owing to muscular volume decreases by withering of the muscle fiber according to aging and by reducing of the number of muscle fibers.
Here, formerly, a muscular weakness by aging has been considered to differ by muscle according to a difference in a daily use situation of the muscles.
However, in recent years, it becomes clear that reduction in muscular volume has happened more in a muscle which supports weight especially.
In particular, in their age of 60's, as for the leg, a decreasing rate of muscular volume is high about 10 to 15% compared with an average of other muscles in whole body.
If lowering of muscular volume in such a muscle that supports weight happens, a possibility of leading to a fall directly will be higher.
Therefore, the necessity of measuring elderly people's muscular volume is rising compared with formerly.
Here, about measurement of muscular volume, there is a method of calculating a muscular quantity from a picture by using a MRI, a CT scan, or the like.
Also, a measuring method of muscular volume by using a body impedance method is established conventionally, and this is more convenient and high-precision.
For example, as technology given in patent documents 1 is referred to, a body composition measuring device which measures and displays muscular volume and bone density of a leg part exists (hereafter, this is called as conventional technology 1.).
[Patent document 1]
JP 2007-7445 A

[Description of the Invention]

[Problem(s) to be Solved by the Invention]

However, in elderly people, the muscular fiber may be replaced with fat, and there is a problem that exact measurement of the muscular volume by MRI or by CT scan is difficult.
On the other hand, the body composition measuring device of conventional technology 1 can measure the muscular volume of the whole body according to right and left by using a plurality of electrodes; however, although the balance of the anteroposterior muscles of a certain part is needed to measure about the muscles which support actual weight, there is a problem that it cannot measure.
This invention is made in view of such a situation, and makes it a subject to cancel an above-mentioned subject.

SUMMARY OF THE INVENTION

A muscular volume balance evaluation apparatus of this invention comprises: at least two sets of electrode pairs which provides a current applying method which applies current to a living body and a voltmeter which measures voltage; the current applying method and the voltmeter are symmetrically located to a specific part of the body; a bioelectricity impedance measuring method which measures bioelectricity impedance of a measured region from current applied by the current applying method and voltage measured by the voltmeter; and a muscular volume calculating method which computes muscular volume from the measured bioelectricity impedance; and it determines for a balance of a muscular volume of a specific part from a computed set of the muscular volume.

A muscular volume balance evaluation apparatus of this invention provides a feature of expandable and contractible longitudinal width, which is an inter-electrode distance located symmetrically with the specific part, and breadth, which is the distance between the electrode pairs.

A muscular volume balance evaluation apparatus of this invention is provided with a longitudinal width measurement methods, which measures the longitudinal width, and a breadth measurement method, which measures the breadth, and is further provided with a volume calculating method which computes volume about muscles to measure by the longitudinal width and the breadth.

A muscular volume balance evaluation apparatus of this invention is that the measurement of balance of the muscular volume is for measuring balance of muscular volume about an anterior surface and a posterior surface of the leg.

A muscular volume balance evaluation apparatus of this invention is further provided with evaluation data in a memory method for evaluating about balance of the muscular volume and computes a desired value of balance of the muscular volume.

A muscular volume balance evaluation apparatus of this invention displays measured muscular volume set separately.

A muscular volume balance valuation method of this invention comprises the steps that: muscular volume is computed from a value of bioelectricity impedance measured in a specific part of the body symmetrically, and balance of muscular volume of a part measured in symmetry is calculated from the computed set muscular volume.

[Effect of the Invention]

According to this invention, the bioelectricity impedance measuring system that can measure the balance of the muscular volume of a specific part can be provided.

BRIEF DESCRIPTION OF THE DRAWINGS

Fig. 1 is an outline view indicating the composition of the muscular volume balance measuring system related to an embodiment of the invention.

Fig. 2 is a figure showing electrode part 230 at in case of measuring bioelectricity impedance related to an embodiment of the invention from the side.

Fig. 3 is a figure showing electrode part 230 at in case of measuring bioelectricity impedance related to an embodiment of the invention from the back.

Fig. 4 is a block diagram showing the control composition of the muscular volume balance measuring system related to an embodiment of the invention.

Fig. 5 is a flow chart showing the measurement procedure and the outline of operation of the muscular volume balance measuring system related to an embodiment of the invention.

Fig. 6 is a conceptual diagram of longitudinal width measurement by longitudinal width measuring part 4a related to an embodiment of the invention.

Fig. 7 is a conceptual diagram of breadth measurement by breadth measuring part 4b related to an embodiment of the invention.

Fig. 8 is a conceptual diagram of another type of longitudinal width measurement by longitudinal width measuring part 4a related to an embodiment of the invention.

Fig. 9 is representative circuit schematic showing bioelectricity impedance.

Fig. 10 is a graph chart showing a bioelectricity impedance vector locus.

Fig. 11 is a conceptual diagram indicating the example of an display as a result of the muscular volume balance measurement related to an embodiment of the invention.

Fig. 12 is a figure indicating the example of an display which points the target except a leg for measuring the muscular volume balance related to an embodiment of the invention.

DESCRIPTION OF THE PREFERRED EMBODIMENTS

[Best Mode of Carrying Out the Invention]

<Embodiment>

(Component of a bioelectricity impedance measuring system)

The suitable embodiment of this invention is described based on a drawing in the following.

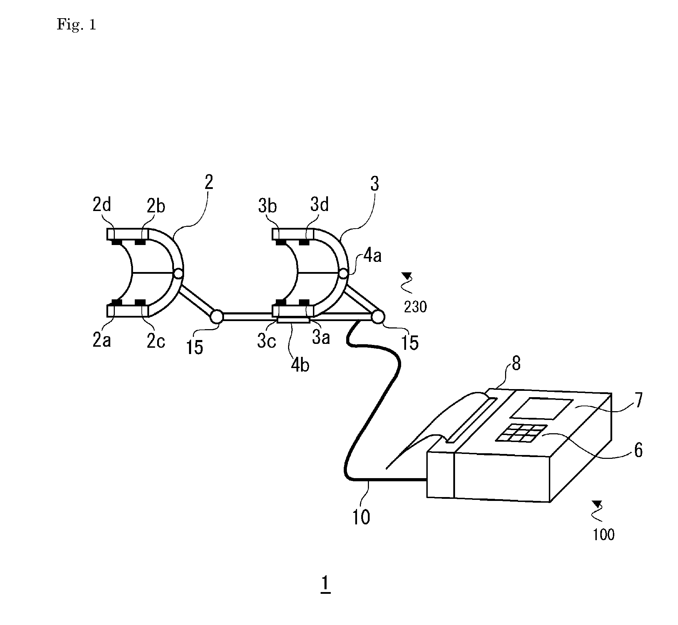

As explained with reference to the outline view of Fig. 1, measuring apparatus 1 of an embodiment of the invention is comprised control part 100 and electrode part 230, and those are connected by code 10.
The upper surface of control part 100 comprises:

input device 6 which is an input method providing a plurality of keys of a power switch and numeric keys and inputting various kinds of body information;
display 7 which is a display method providing dot matrix LCD, an organic electroluminescence panel, or the like, which displays a measurement result; and
printing equipment 8 which prints and ejects a measurement result in a paper.

(Composition of an electrode)

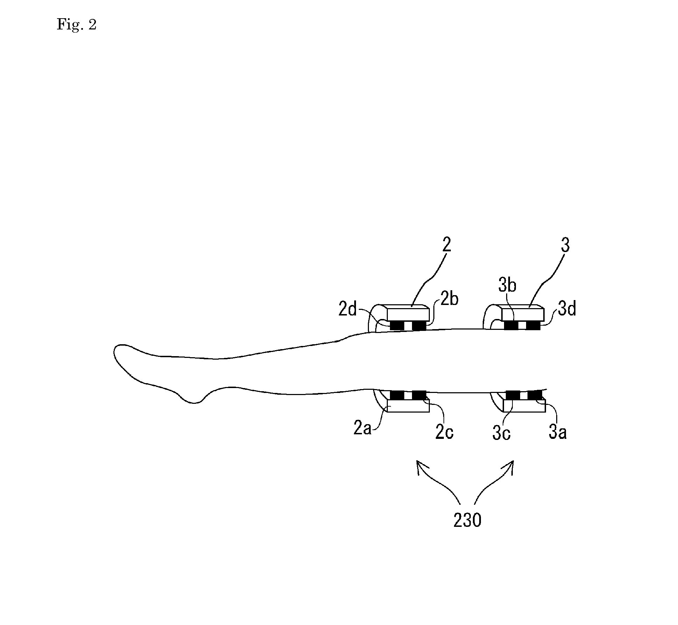

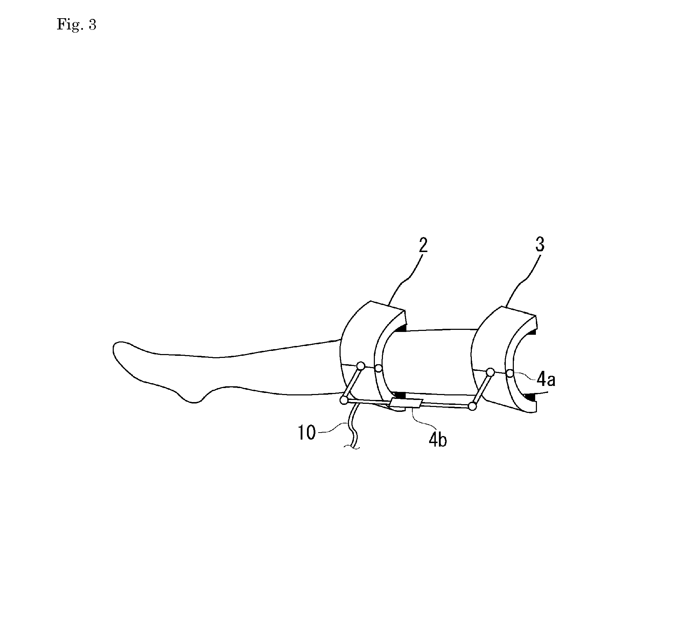

Further in reference to Fig. 2 and Fig. 3, an example of measuring in case of a situation that a being-measured person having lain down to upward is explained.
Moreover, this measuring apparatus 1 can also measure in the proneness, the standing posture, or the sitting-down posture, in addition to the facing up posture.
Electrode parts 230 are a current applying method and a voltmeter; and it is comprised connecting clip 2 and clip 3 (electrode pair) with joint part 15, which is adjustable a direction of each clip.
As for clip 2 and clip 3, it is provided current supply source electrodes 2d and 3d and voltage measurement electrode 2b and 3b, which touch the upper part (it becomes the upper part when a being-measured person measures in the state where he or she lay down upward) of a specific part to measure, and it is also provided current supply source electrodes 2a and 3a and voltage measurement electrodes 2c and 3c, which touch the lower part of the part to measure. These electrodes are connected symmetrically (such as inserting) to the specific part to measure.
These electrodes are connected to electrode switching device 20 via code 10.
Also, clip 2 can freely adjust, for example, a longitudinal width, which is the distance between electrodes 2a and 2d.
Similarly, clip 3 can also freely adjust, for example, a longitudinal width which is the distance between electrode 3a and electrode 3d.
Further, length (distance) between clip 2 and clip 3 is variable, which is able to expand and contract.
Here, electrode part 230 provides:

Longitudinal width measuring part 4a (longitudinal width measurement method) which is an encoder, slide variable resistor, resistant arrays, or the like, put inside of clip 2 or clip 3 and measures inter-electrode longitudinal width, and
Breadth measuring part 4b (breadth measurement method) which is an encoder, slide variable resistor, resistant arrays, or the like, provided in joint part 15 and is used in order to measure breadth.

(Internal configuration)

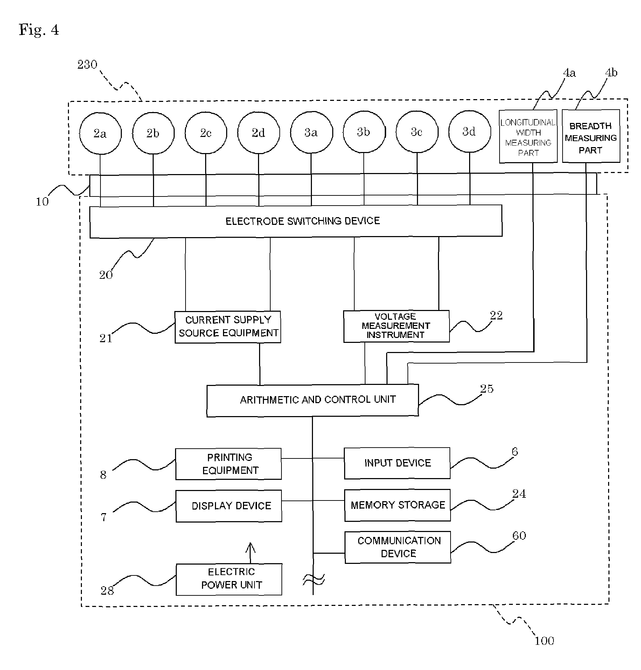

Control composition of measuring apparatus 1 is explained further with reference to a block diagram of Fig. 4.
In inside, control part 100 provides:

Memory storage 24 comprises memories, such as RAM or ROM which is a memory method memorizing various data or the like, and auxiliary storages, such as a flash memory and HDD;
Arithmetic and control unit 25 comprises a microcomputer (CPU) and a bioelectricity impedance calculating method that computes bioelectricity impedance from applied current and measured voltage;
Electric power unit 28 supplies electric power to arithmetic and control unit 25 or each other equipment;
USB (universal serial bus) can connect with other computers which are PCs (personal computer) or the like; and
Communication device 60 provides, such as, terminals of LAN (local area network), wireless LAN, Bluetooth (registered trademark), connecting apparatus of wireless USB, or the like.

Arithmetic and control unit 25 is also a compensation method that correct computed bioelectricity impedance.
It is also a body composition calculating method that computes index(s) about a living body's composition, and it can carry out about the other various kinds of operations and control.
Arithmetic and control unit 25 is also connected with input device 6, display 7, and printing equipment 8.
From electrode part 230, code 10 linked to each electrode, longitudinal width measuring part 4a, and breadth measuring part 4b, connects with control part 100.
A signal wire from each electrode in code 10 is connected to current supply source equipment 21 and voltage measurement instrument 22 via electrode switching device 20 which is an electronic relay circuit, or the like.
This current supply source equipment 21 and voltage measurement instrument 22 are connected to arithmetic and control unit 25, which is a control method.

A signal wire linked to longitudinal width measuring part 4a of code 10 and breadth measuring part 4b is directly connected to I/O of arithmetic and control unit 25, or the like.

(Measurement of muscular volume)

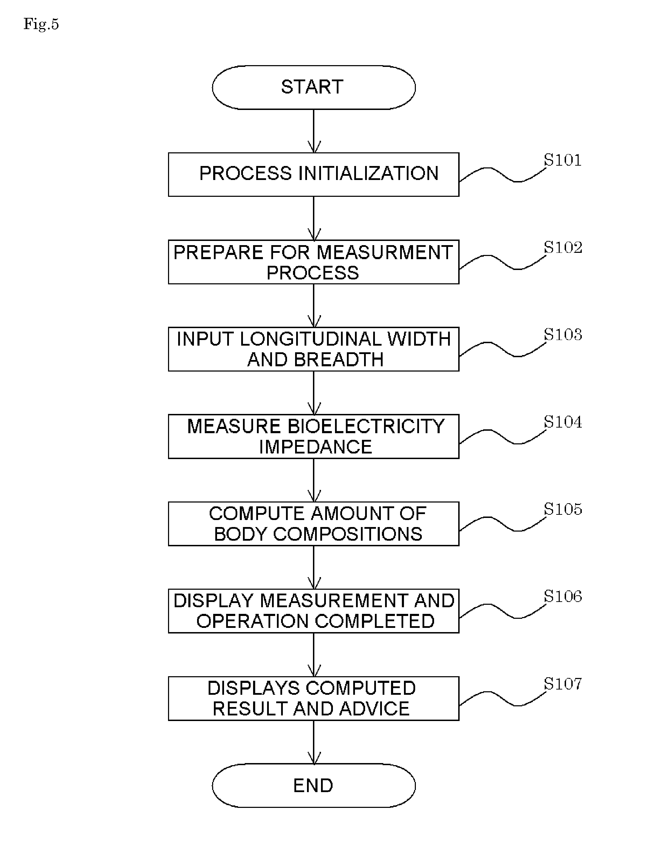

Then, as refer to Fig. 5, which is a flow chart, measurement of muscular balance related to an embodiment of the invention is explained.

At first, if measuring person pushes the switch (not illustrated,) electric power is supplied to each part from electric power unit 28.

Thereby, arithmetic and control unit 25 starts, and it begin to execute firmware memorized in ROM or a flash memory of memory storage 24, or the like.

(Step S101)

Then, in Step S101, arithmetic and control unit 25 carries out initialization processing recorded in firmware.

Thereby, arithmetic and control unit 25 initializes each part and displays an initial screen, which is not illustrated, on display 7.

When initialization processing of each part is completed, arithmetic and control unit 25 advances processing to the following step S102.

(Step S102)

In Step S102, arithmetic and control unit 25 displays having ended starting process and having carried out preparation-for-measurement completion on display 7.

Here, measuring person or being-measured person inputs individual parameters, such as sex, age, and height.

By using a number key of input device 6 or the like, it is carried out an input of this individual parameter.

It may input by other computers via communication device 60.

About data of this individual parameter, arithmetic and control unit 25 memorizes to RAM of memory storage 24.

Then, as shown in Fig. 2 and Fig. 3, measuring person attaches clip 2 and clip 3 of electrode part 230 of this measuring apparatus 1 so that a specific part (in this case, thigh) which measures balance of muscular volume of a being-measured person may be inserted and makes them contact.

In an embodiment of the invention, a case where it measures about balance of muscular volume of the anterior surface and the posterior surface (if measuring upward, up-and-down) of the thigh is explained.

Measuring person aligns a direction to be desired to measure balance of muscular volume (in this case, the knee side and the sural-region side) with a direction of sandwiching the clips (the up-and-down direction).

Here, current supply source electrodes 2d and 3d and voltage measurement electrode 2b and 3b are knee sides, and current supply source electrodes 2a and 3a and voltage measurement electrodes 2c and 3c become the sural-region side.

Then, measuring person's pressing a key of input device 6 interactively and directing a measurement start by instructions of a menu displayed on display 7, according to an indication of display 7, start an actual measuring process.

(Step S103)

In Step S103, arithmetic and control unit 25 measures an opening width (hereafter, it is called as longitudinal width) of any one of clip 2 or 3 based on a signal of longitudinal width measuring part 4a; and a distance (hereafter, it is called as breadth) between electrode 2c and electrode 3c or between electrode 2b and electrode 3b, which is the distance between clip 2 and clip 3, is measured based on a signal of breadth measuring part 4b.

Arithmetic and control unit 25 memorizes these longitudinal width and breadth to RAM of memory storage 24, and in the following step, when computing each muscular volume of the upper part and the lower part from a value of bioelectricity impedance, they are used in order to compute volume.

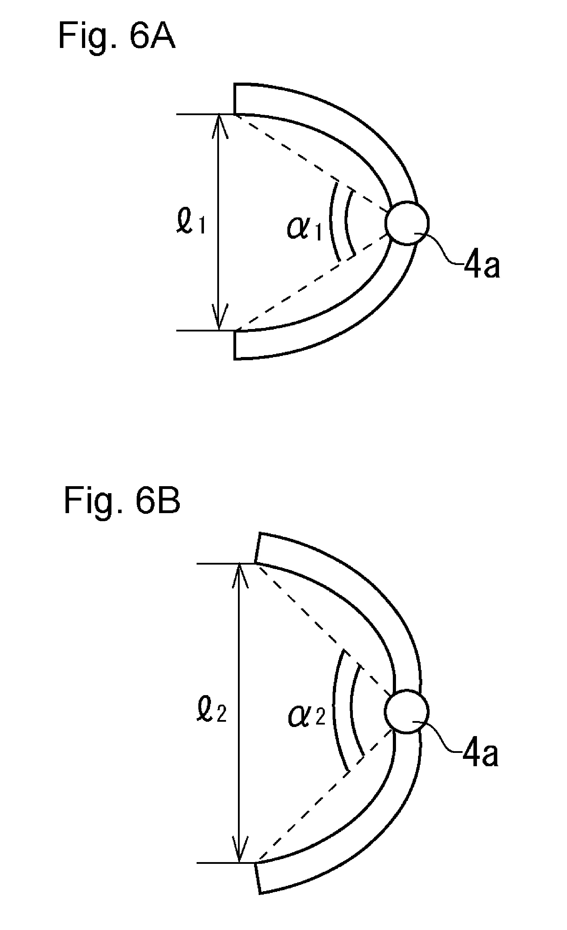

In the following, with reference to Figs. 6 - 8, it explains in detail.

As refer to Fig. 6, for example, longitudinal width measuring part 4a will measure distance 1 which a clip opens from opening-and-closing angle alpha of the clip by using a rotary encoder.

Specifically, binary data of a Gray code corresponding to angle alpha is inputted into I/O of arithmetic unit 25 by using a rotary encoder of an absolute system.

This value is compared with a value in ROM in arithmetic and control unit 25, and distance 1 corresponding to inputted alpha value is determined.

In the case of small angle alpha, $alpha_1$ (Fig. 6A), distance 1 serves as small value $l_1$; and, in the case of large angle

alpha, alpha$_2$ (Fig. 6B), distance l serves as large value l$_2$.

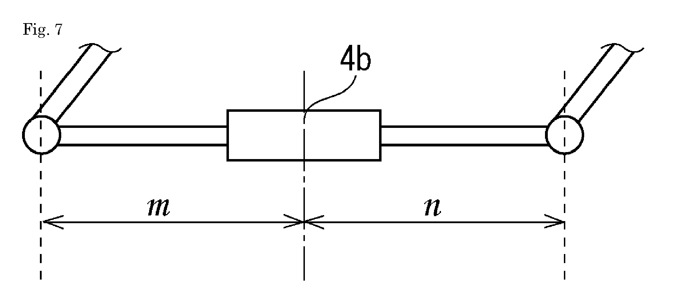

As refer to Fig. 7 similarly, for example, breadth measuring part 4b measures distance m and n between clips by using a linear encoder.

Specifically, binary data of a Gray code corresponding to distance m and n for an absolute system can be inputted into I/O of direct arithmetic unit 25.

Distance m and n are calculated by comparing these values with values in ROM of arithmetic and control unit 25.

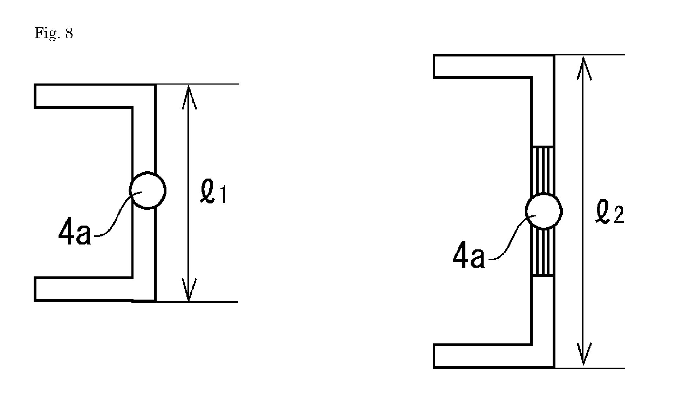

Otherwise, as Fig. 8 is referred to, in clip 2 and clip 3, it is also enabled to use a clip of moving up and down method and inserting the specific part instead of rotating and inserting method for the specific part.

In this case, a linear encoder can be used for longitudinal width measuring part 4a.

Thereby, the distance $1 = l_2 - l_1$ that the clips open is measurable with elastic distance as well as the example of breadth measuring part 4b.

As for the above-mentioned encoder, naturally, it is also enabled to use an encoder of an incremental system.

(Step S104)

Then, in Step S104, arithmetic and control unit 25 measures bioelectricity impedance (living body BI).

In the following, we explain that in this case of carrying out the operation of bioelectricity impedance measurement (multifrequency body impedance measurement) by using AC of a plurality of frequency.

Multifrequency bioelectricity impedance measurement starts the frequency Fi from i = 1 in applied current and carries out n times set up.

As initialization of measurement in the first frequency, i = 1 is set up, and frequency Fi is set up with a value of this i.

Based on a measurement control parameter located previously at ROM in memory storage 24 (it is hereafter called as a measurement control parameter), arithmetic and control unit 25 sets up output signal frequency, and outputs the output signal to current supply source equipment 21.

Current supply source equipment 21 comprises a constant current output circuit which can set up a current value, and sets up an output current value based on a measurement control parameter.

When measuring the upper part (in this case, the knee side) firstly, AC output applies current to a being-measured person via electrode switching device 20 with current supply source electrodes 2d and 3d which touch the upper part.

At this time, voltage is measured by voltage measurement electrode 2b and 3b.

Subsequently, when measuring the bottom (in this case, the sural-region side), current is applied to a being-measured person via electrode switching device 20 with current supply source electrodes 2a and 3a that touch the lower part.

At this time, the voltage is measured with voltage measurement electrodes 2c and 3c.

At the time of this measurement a current which flows into a being-measured person from electrodes 2a and 3a or electrodes 2d and 3d is detected by reference resistance (not illustrated) provided inside of power supply unit 21; an analog signal of the output is converted into a digital signal by using an A/D converter in arithmetic and control unit 25; and the result is memorized to RAM of arithmetic and control unit 25.

Simultaneously, voltage generated among Electrode 2b, 3b or electrode 2c, 3c are inputted into a differential amplifier circuit in voltage measurement instrument 22 through a voltage measurement electrode in contact with the being-measured person at this time; and a differential amplifier circuit outputs a signal which is a difference of each inputted voltage to an A/D converter in arithmetic and control unit 25.

An A/D converter measures bioelectricity impedance by converting an inputted analog signal into a digital signal, and memorizes the result into RAM.

If impedance measurement by the first frequency is completed, 1 is added to i, and it is judged whether a regular number of determinations is ended.

Here, if i is over n, which is a predetermined number, the measurement of impedance is ended; and if it has not yet exceeded, it will carry out impedance measurement in the following frequency.

Then, from bioelectricity impedance measured value measured in step S104, a bioelectricity impedance vector locus and parameter(s) about it is computed.

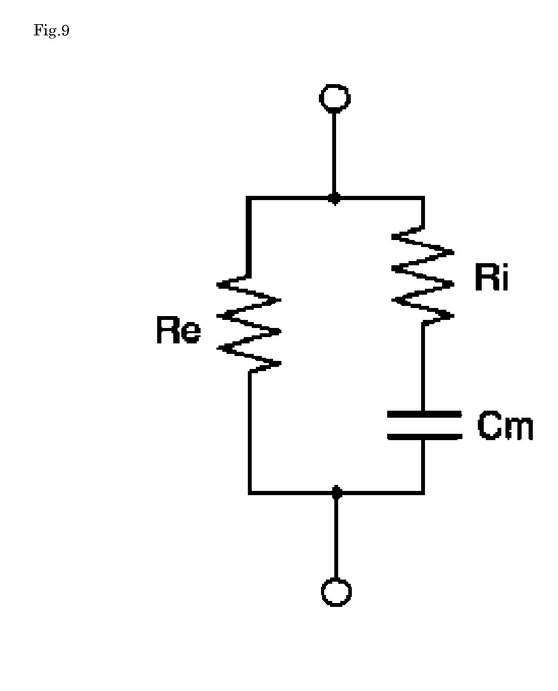

Usually, bioelectricity impedance is shown in an equivalent circuit which described in concentrated constants of extracellular fluid resistance Re, intracellular fluid resistance Ri, and cell membrane capacity Cm, as shown in Fig. 9; however, in fact, since each cell which comprises a living body is shown in a circuit where constants are differ as the shape and character difference, respectively; and in a living body which is the totality, a bioelectricity impedance vector locus does not serve as a semicircle, such as, in the case of measuring an equivalent circuit by a concentrated constant, and it is supposed that it becomes a circle according to a Cole-Cole circular arc law.

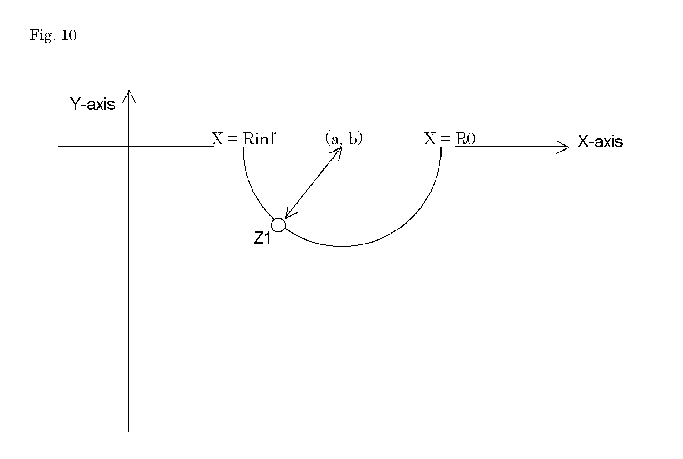

Therefore, generally, bioelectricity impedance draws a circular locus as shown in Fig. 10.

Here, the X-axis shows a resistance component of bioelectricity impedance, and a Y-axis shows a reactance component of bioelectricity impedance.

Because a reactance component of bioelectricity impedance is capacitive, it takes a minus value, and a bioelectricity impedance vector locus is located under the X-axis; and also from assumption that a bioelectricity impedance vector locus to determine is a circle, in each of Frequency F1, F2, --, FN, bioelectricity impedance measured value Z1, Z2, --, ZN are on the circumference of a certain circle.

Here, when X coordinate of the center of a circle is "a," Y coordinate of the center of the circle is "b," radius of the circle is "r," an equation of the circle which passes along bioelectricity impedance measured value is shown, such as, in equation 1.

$$(X - a)^2 + (Y - b)^2 = r^2 \quad \dots \text{(equation 1)}$$

a, b, and r are calculated by substituting bioelectricity impedance measured value Z1, Z2, --, ZN in frequency F1, F2, --, FN, respectively, to the equation 1.

Also, by equations 1, X are expressed as follows.

$$X = a \pm \text{sqrt}(r2 - b2) \dots \text{(equation 2)}$$

sqrt() shows a square root.

Then, R0 and Rinf (R0>Rinf), which are the intersections of a circle and the X-axis indicated in equation 1, are calculated by equation 2 as follows.

$$R0 = a + \text{sqrt}(r2 - b2) \quad \dots \text{(equation 3)}$$

$$\text{Rinf} = a - \text{sqrt}(r2 - b2) \quad \dots \text{(equation 4)}$$

Further, by equation 3 and equation 4, Re and Ri in an equivalent circuit of Fig. 9 are calculated as follows.

$$\text{Re} = R0 \quad \dots \text{(equation 5)}$$

$$\text{Ri} = R0 * \text{Rinf} / (R0 - \text{Rinf}) \quad \dots \text{(equation 6)}$$

Because bioelectricity impedance vector Zc in characteristic frequency Fc is a reactance component, that is, the point that an absolute value of Y axial component becomes the maximum, X coordinate value, which is a resistance component in that case, and a Y coordinate value, which is reactance components, are computed as follows.

$$X = a \quad \dots \text{(equation 7)}$$

$$Y = b - r \quad \text{.... (equation 8)}$$

Here, when Rc is a resistance component of Zc, and Xc is a reactance component of Zc; and Zc is expressed as follows.

$$Zc = Rc + j * Xc = a + j(b - r) \quad \text{.... (equation 9)}$$

Also, when Z(omega) is a bioelectricity impedance vector in omega and tau and beta are constant(s), a bioelectricity impedance vector in any angle of frequency omega is shown as follows by Cole-Cole circular arc law.

$$Z(omega) = (R0 - Rinf) / \{1 + (j * omega * tau) * beta\} \text{....}$$

$$\text{(equation 10)}$$

Furthermore, equation 10 is shown as follows as tau = 1 /(omega * c).

$$Z(omega) = (R0 - Rinf) / \{1 + ((j * omega) / (omega * c)) *$$

$$beta\} \text{.... (equation 11)}$$

Here, since omega * c = 2 * pi * Fc, Fc and beta are calculated by using bioelectricity impedance measured value measured previously.

(Step S105)

Then, in Step S105, arithmetic and control unit 25 computes the amount of body compositions and memorizes to RAM of memory storage 24.
This is, based on a bioelectricity impedance vector locus to which is calculated by bioelectricity impedance measured value and parameter about it, as mentioned above, R0 and Rinf, Re and Ri, Zc, Rc, Xc, Fc, or the like, the amounts of body compositions, such as, amount of body fat and a fat-free mass (difference of weight and amount of body fat), are computed.
Also, values, such as intracellular fluid volume ICW, extracellular fluid volume ECW, intracellular /extracellular fluid volume ratio ICW/ECW, and the total body water TBW (= ICW + ECW; the sum of extracellular fluid volume and intracellular fluid volume), can also be calculated from the computed amount of body compositions.
For example, values of intracellular fluid volume ICW, extracellular fluid volume ECW, and the total body water TBW are calculated by the following equations by using values of Ri, Re, height Ht, and weight W.

$$ICW = Ki1 * Ht2 / Ri + Ki2W + Ki3$$

$$ECW = Ke1 * Ht2 / Re + Ke2W + Ke3$$

$$TBW = ICW + ECW$$

(Where, Ki1, Ki2, Ki3, Ke1, Ke2, and Ke3 are the coefficients.)

Also, arithmetic and control unit 25 computes muscular volume by the muscular volume measurement program (muscular volume measuring method) memorized in ROM of memory storage 24.

At first, it calculates for volume of a measured region from above-mentioned longitudinal width and breadth.

The volume is computed by a volume measurement routine (volume calculating method) of a muscular volume measurement program.

In this case, for example, it is adopted a model that the volume through current flowing into the inside of the body is in proportion to volume of a rectangular parallelepiped, and it is computed by the following formulas:

$$Volume = longitudinal\ width * breadth * vh$$

(In the above, vh is a coefficient)

In measuring apparatus 1 related to an embodiment of the invention, regression based on Rc corresponding to volume and muscular volume is prepared, and muscular volume is computed by applying Rc, which is the measured value related to an embodiment of the invention, for the upper part or the lower part.

Regression or a data table by this correlation equation is memorized in ROM of memory storage 24.

In this equation, for example, it is available by using a function corresponding to a value of volume, age, and bioelectricity impedance in the following:

$$M(muscular\ volume) = f(volume,\ age,\ bioelectricity$$

$$impedance\ value)$$

(Step S106)

Subsequently, in Step S106, arithmetic and control unit 25 displays on display 7 that measurement and operation are completed.

(Step S107)

Subsequently, in Step S107, arithmetic and control unit 25 displays muscular volume on display 7, and carries out advice to the subject.

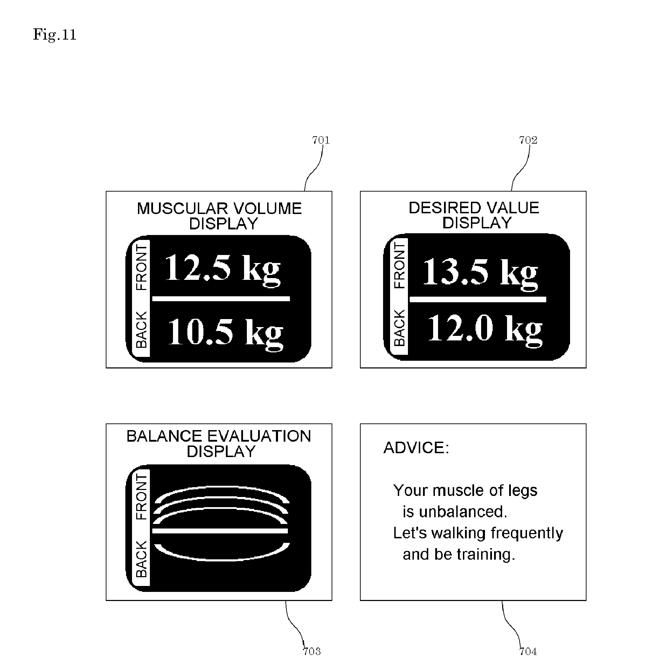

With explaining as refer to Fig. 11, when measuring person carry out the push-down of the key of input device 6, it can be changed that display screen 701 which displays muscular volume, display screen 702 which displays muscular volume of a desired value of muscular volume calculated with an individual parameter of a being-measured person, and display screen 703 which displays balance of muscular volume graphically.

In these display screens, muscular volume measured with an upside electrode and muscular volume measured with a lower electrode are displayed as front and back, respectively.

In display screen 701, muscular volumes determined by the above-mentioned measurement and calculation of muscular volume is displayed.

In display screen 702, based on sex, age, height, or the like, standard muscle mass, in the case of people of the same sex, the same age, and the same height, is calculated from evaluation data of muscular volume balance and regression for evaluation, which is memorized in memory storage 24, and they are displayed as desired values.

In display screen 703, it can express, for example, with the number of lines such as a bow which is imitated muscles by a ratio of muscular volume of display screen 701 and muscular volume of display screen 702.

By measuring person being carried out the push-down of the key of input device 6, display screen 704 which displays advice of a measurement result can be displayed.

In this advice, it displays a text of the ratio of muscular volume of above-mentioned display screen 701 and muscular volume of display screen 702, which is memorized in ROM of memory storage 24 and is based on a threshold value for the ratio of the front and the back.

When measuring person carry out the push-down of the key of input device 6, the contents of display screens 701-704 and the measured value which includes muscular volume, fat rate, bone density, or the like, from above-mentioned measurement results, are printed by printing equipment 8.

At the time of this printing, it is also enabled to print a document of more detailed advice.

Transmission to other computers can be carried out for these measurement results by using communication device 60.

In this case, checking about a measurement result is enable on a screen of the other computer.

When a measurement result is confirmed, measuring person turns off this measuring apparatus 1, and the measurement is completed.

Measuring apparatus 1 related to an embodiment of the invention can provide bioelectricity impedance measuring apparatus that can measure front and back in a specific part, that is, front and back muscular volume.

By a measurement result, muscular balance of front and back in a certain part can be measured on the occasion of reduction of muscular volume by aging in muscle that supports actual weight.

In the case of an athlete, when balance of muscle mass is bad, injury will be occurred, and there is a possibility of lowering the performance.

Against this, it becomes enabled by measuring balance of anteroposterior muscular volume of muscular volume by using this measuring apparatus 1 to prevent an injury for an athlete.

In this case, muscle mass which serves as a characteristic standard according to a kind of sport is memorized, and it can judge individually according to a kind of sport.

Also, in the case that an athlete actually causes injury, such as a pulled muscle, and is carrying out rehabilitation, it can be used, such as, when there is no telling how much have recovered, or when he or she desires to gain good muscular volume of anteroposterior balance so that it may not recur after recovering.

Thereby, an athlete's performance can be raised, and muscular volume being not caused injury easily can be obtained.

This measuring apparatus 1 does not only measure lowered muscular volume according to aging but also measure about a balance transition of muscles when muscular volume is recovering after reducing the muscular volume by a certain cause.

For example, since activity of a muscle will decline over a long period of time when cast immobilization is made by fracture, a muscle shows large withering.

In this case, specific muscle at fixed place shows higher withering degree.

Therefore, for example, it becomes enabled to measure convenient about recovery at the case of removing gibbs and carrying out rehabilitation.

Also, when stay in space a long period of time, withering of a muscle arises.

An astronaut, who stays long term in a space station or the like, is carrying out exercise every day in order to prevent withering of a muscle and is aiming at maintenance of bone density.

However, balance of muscular volume might be spoiled depending on how to cover load of exercise, and a method of recognizing balance of muscular volume is demanded.

Here, balance of an astronaut's muscular volume can be measured with this measuring apparatus 1 in the aerospace.

Furthermore, in the state of a free fall state or very small gravity, it is known that distribution of water in the living body will change.

For this reason, when this measuring apparatus 1 measures muscular volume in aerospace, measuring person need to measure by using input device 6 and sets it as "space mode" which corrects an equation of calculation of muscular volume interactively, in advance.

Although described with control part 100 and electrode part 230 having separated in measuring apparatus 1 related to an embodiment of the invention, it may be provided with a circuit equivalent to control part 100, which is inner part of clip 2 or 3 or unites with clips 2 and 3, and an indication and input device, which is a liquid crystal device with a touch panel, or the like, in the opposite side of an electrode of a clip.

In this case, because it can measure with the equipment which is comprised of a single part, transportability improves.

Since it is not dependent on the length of code 10, flexibility of measuring operation of measuring person or being-measured person can be extended more in the case of measurement.

Although it explained that measuring apparatus 1 related to an embodiment of the invention stuck an electrode with a clip, as a matter of course, an electrode which is directly sticking on the skin may be used.

In this case, when measuring person view methods, such as flexible measure, inter-electrode longitudinal width and breadth can be inputted.

Furthermore, an electrode of a type twisted around a specific part can also be used.

In an above-mentioned embodiment of the invention, an example which measures balance of muscular volume of a leg is explained; however, if it is a part desired to measure balance of muscular volume or a part desired to measure muscular volume of specific muscles, measuring anywhere in the body is enabled.

In this case, a measurement point based on an anatomical chart is prepared, and when a part which measuring person want to measure is inputted with input device 6, arithmetic and control unit 25 displays a measurement point along a figure of human body on display 7.

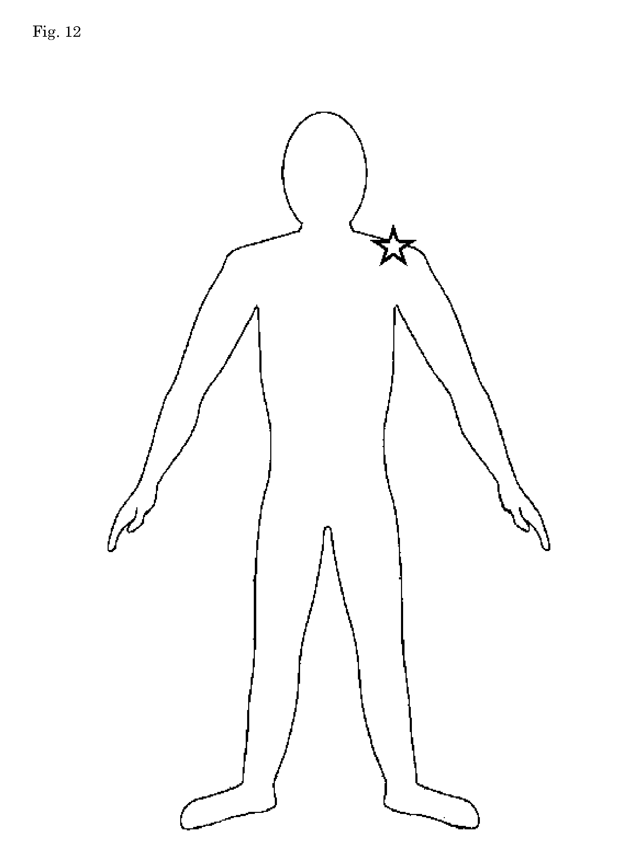

For example, as refer to Fig. 12, it shows a star mark in a position of the shoulder.

When measuring person attach clip 2 and clip 3 to a being-measured person according to instructions of this display 7, for example, balance of muscular volume of the shoulder triggering the shoulder discomfort can be measured.

Also, it is extremely important for an athlete to get to know of which position muscular volume is increased.

For this reason, this measuring apparatus 1 can also be used for such as a swimmer, a sprinter, a long-distance runner, as for example, where a utilization of measuring the muscles that is suitable for each sport increased by training.

Furthermore, for an embodiment of the invention, although the electrode are considered as two sets of upper and lower sides, muscular volume and balance of a specific part can be more accurately measured by using a number more than these, such as an electrode array or the like.

This measuring apparatus 1 can be transmitted a measurement result to the other computers which is not illustrated, since the other computers are provided with nonvolatile memory measures, which is an auxiliary storage such as HDD, it can carry out checking data of this sequential observation conveniently.

Since measurement of muscular volume is convenient, it is suitable for pursuing a change with time of a state of muscular volume; and since a numerical value can increase with recovery and it can be ascertained visually, it is enabled to promote volition of a being-measured person by applying to recovery or a measuring person by recovery.

In addition, unlike a bioelectricity impedance measuring device of conventional technology 1, this measuring apparatus 1 has memorized regression of a subject who includes a child and an old person in ROM of memory storage 24.

For this reason, it can measure accurately also about muscular volume of a child or an old person.

Therefore, it is enabled to ascertain the restorative degree more accurately.

Composition and operation of the above-mentioned embodiment are an example, and it is needless to say that it can change suitably and can execute in the range that does not deviate from the purport of this invention.

[Reference Numerals]

| | |
|---|---|
| 1 | Measuring apparatus |
| 2 | and 3 Clip |
| 2a, 2d, 3a, and 3d | Current supply source electrode (current applying method) |
| 2b, and 2c, 3b and 3c | Voltage measurement electrode (voltmeter) |
| 4a | Longitudinal width measuring part |
| 4b | Breadth measuring part |
| 6 | Input device |
| 7 | Display device |
| 8 | Printing equipment |
| 10 | Code |
| 15 | Joint part |
| 20 | Electrode switching device |
| 21 | Current supply source Equipment |
| 22 | Voltage measurement instrument |
| 24 | Memory storage |
| 25 | Arithmetic and control unit |
| 28 | Electric power unit |
| 60 | Communication device |
| 100 | Control part |
| 230 | Electrode part |
| 701, | 702, 703, and 704 Display screen |

**Claims**

1. A muscular volume balance evaluation apparatus, which comprises:

   at least two sets of current applying method and voltmeter are symmetrically located to a specific part of the body;
   a bioelectricity impedance measuring method measures bioelectricity impedance of a measured region from current applied by the current applying method and from voltage measured by the voltmeter;

a muscular volume calculating method computes muscular volume from the measured bioelectricity impedance; and

the muscular volume balance evaluation apparatus determines for balance of muscular volume in the specific part by the computed set of the muscular volume.

2. The muscular volume balance evaluation apparatus according to claim 1, wherein
longitudinal width, which is an inter-electrode distance symmetrically located to the specific part, and
breadth, which is the distance between the electrode pairs,
are able to expand and contract.

3. The muscular volume balance evaluation apparatus according to claim 1 or 2, which further comprises:

a longitudinal width measurement method measures the longitudinal width;
a breadth measurement method measures the breadth; and
a volume calculating method computes volume for the muscle to measure by the longitudinal width and the breadth.

4. A muscular volume balance evaluation apparatus according to any one of the claims 1 to 3, wherein the measurement of balance of the muscular volume is for measuring balance of muscular volume about anterior surface and posterior surface of leg.

5. A muscular volume balance evaluation apparatus according to any one of the claims 1 to 4, which further comprises evaluation data in a memory method to evaluate about balance of muscular volume, and computes a desired value of balance of the muscular volume.

6. A muscular volume balance evaluation apparatus according to any one of the claims 1 to 5, wherein each of the muscular volume for the computed set is displayed separately.

7. A muscular volume balance valuation method, comprising the steps of:

computing muscular volume from a value of bioelectricity impedance measured in a specific part of the body; and
calculating balance of muscular volume of the specific part measured by the computed set of muscular volume.

Fig. 1

1

Fig. 2

Fig. 3

Fig. 4

Fig.5

START

PROCESS INITIALIZATION — S101

PREPARE FOR MEASURMENT PROCESS — S102

INPUT LONGITUDINAL WIDTH AND BREADTH — S103

MEASURE BIOELECTRICITY IMPEDANCE — S104

COMPUTE AMOUNT OF BODY COMPOSITIONS — S105

DISPLAY MEASUREMENT AND OPERATION COMPLETED — S106

DISPLAYS COMPUTED RESULT AND ADVICE — S107

END

Fig. 6A

Fig. 6B

Fig. 7

4b

$m$

$n$

Fig. 8

4a  $\ell_1$

4a  $\ell_2$

Fig.9

Fig. 10

Fig.11

701

**MUSCULAR VOLUME DISPLAY**

FRONT
**12.5 kg**
BACK
**10.5 kg**

702

**DESIRED VALUE DISPLAY**

FRONT
**13.5 kg**
BACK
**12.0 kg**

**BALANCE EVALUATION DISPLAY**

FRONT

BACK

703

ADVICE:

Your muscle of legs
is unbalanced.
Let's walking frequently
and be training.

704

Fig. 12

**European Patent Office**

**EUROPEAN SEARCH REPORT**

Application Number

EP 08 15 7817

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/059242 A1 (MASUO YOSHIHISA [JP] ET AL) 25 March 2004 (2004-03-25) | 1,2,4-7 | INV. A61B5/053 |
| Y | * abstract; figures 1,2,31,46 * <br> * paragraph [0015] * <br> * paragraphs [0216] - [0218] * <br> * paragraph [0266]; figure 21 * <br> * paragraphs [0252], [0253] * <br> * paragraphs [0275], [0278] * <br> ----- | 3 | |
| X | US 2003/216665 A1 (MASUO YOSHIHISA [JP] ET AL) 20 November 2003 (2003-11-20) <br> * abstract; figures 5-8 * <br> * figures 26-36 * <br> * paragraphs [0031], [0060], [0241] * <br> * paragraphs [0257] - [0279] * <br> ----- | 1-7 | |
| Y | JP 2004 351135 A (JAPAN SCIENCE & TECH AGENCY; MOROE TERUYOSHI; IGARASHI AKIRA; UNIV KAN) 16 December 2004 (2004-12-16) <br> * abstract; figure 1 * <br> ----- | 3 | |

TECHNICAL FIELDS SEARCHED (IPC)

A61B

The present search report has been drawn up for all claims

| Place of search | Date of completion of the search | Examiner |
|---|---|---|
| Munich | 29 August 2008 | Daniel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 08 15 7817

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2008

| Patent document cited in search report | | Publication date | Patent family member(s) | | Publication date |
|---|---|---|---|---|---|
| US 2004059242 | A1 | 25-03-2004 | AU | 1849602 A | 11-06-2002 |
| | | | CN | 1489447 A | 14-04-2004 |
| | | | WO | 0243586 A1 | 06-06-2002 |
| | | | JP | 4124649 B2 | 23-07-2008 |
| US 2003216665 | A1 | 20-11-2003 | CN | 1494869 A | 12-05-2004 |
| | | | JP | 4105472 B2 | 25-06-2008 |
| | | | JP | 2003299629 A | 21-10-2003 |
| JP 2004351135 | A | 16-12-2004 | JP | 3880547 B2 | 14-02-2007 |

EPO FORM P0459

For more details about this annex : see Official Journal of the European Patent Office, No. 12/82

**EP 2 016 896 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007007445 A